# EUROPEAN PATENT APPLICATION

(11) **EP 2 000 483 A2**
(43) Date of publication of application: **10.12.2008**
(21) Application number: 07720595.3
(22) Date of filing: 29.03.2007
(51) Int. Cl.: C07K 19/00, C12N 15/62, C12N 15/63, A61K 38/17, A61P 27/02

(54) **VEGF RECEPTOR FUSION PROTEIN AND USE THEREOF**

(30) Priority: 31.03.2006 CN 200610066257
(71) Applicant: Chengdu Kanghong Biotechnologies Co., Ltd., Chengdu City 610036, Sichuan (CN)
(72) Inventor: YU, Decao Michael, Chengdu City 610036, Sichuan (CN)
(74) Representative: Predazzi, Valentina
(86) International application number: PCT/CN2007/001021
(87) International publication number: WO 2007/112675

(57) **Abstract**

Vascular endothelial growth factor (VEGF) receptor fusion protein comprising Ig domain 2 of Flt-1 and Ig domains 3, or Ig domain 2 of Flt-1 and Ig domain 3 and 4 of KDR, the gene encoding the fusion protein, the pharmaceutical composition containing the fusion protein and the pharmaceutical use of the fusion protein are provided. The fusion protein can be used for treatment of eye disorders involving angiogenesis such as diabetic retinopathy.

## Description

### FIELD OF INVENTION

The present invention relates to VEGF receptor fusion proteins, the pharmaceutical compositions comprising the same, and related therapeutic applications for various eye diseases caused by angiogenesis.

### BACKGROUND OF INVENTION

Retinal vessels and chorodial vessels are the essential components of the retina. Abnormal changes in the vessel wall structure and function of the blood vessels caused by traumas and diseases are the main factors that lead to a hypopsia and visual loss. For example, diabetic retinopathy, which is caused by diabetes mellitus that result in hyperplasia in retinal vessels and detachment of the retina thereafter, is the main factor that leads to the visual loss. The retinal neovascularization might also occur during recovery from an eye injury or surgery. This kind of hyperplasia in retinal vessels is also a key factor that leads to a hypopsia or visual loss (Nature 438: 932-938, 2005).

The age-related macular degeneration (AMD) is a disease caused by the cells or tissue degradation and vascular proliferation occurred in the center area of the retina. It may be classified into the wet form (exudative form) and the dry form. The wet AMD is most commonly caused by choroidal neovascularization and is also the main factor that leads to visual loss.

Vascular endothelial cell growth factor (VEGF) is a protein that specifically mediates angiogenesis (Am. J. Pathol. 167: 1451-1459, 2005). VEGF stimulates division and proliferation of the endothelial cells, induces onset of neovascularization, and provides oxygen and nutrition to the tissue cells. Many studies have shown that once photoreceptor cells of the retina start to degenerate (ischemic atrophy) because of lack of nutrition, the concentration of VEGF in the retina starts to increase to promote the neovascularization. This process is called angiogenesis. In the eyes, the newly generated blood vessel is different from the normal blood vessel in morphology, the vessel lumen is irregular and the tube wall is often leaky. This kind of abnormal growth of the highly permeable or leaky blood vessels often results in scars in the retina, and even detachment, which in turn affects the eyesight.

Many studies have shown that the choroidal tissues of the wet AMD patients exhibit high level of VEGF expressions (Invest. Opthal. Vis. Sci 37: 855-868, 1996; Microvascular Res. 64: 162-169, 2002). Considering the correlation in between the level of VEGF expressions and the wet AMD, VEGF can be used as a biochemical indicator in the AMD diagnosis (Br. J. Opthalmol. 88: 809-815, 2004).

Some VEGF inhibitors can block the interactions between VEGF and the VEGF receptors (flt-1, KDR, etc) on the endothelia cell, and thus block the signal transduction mediated by VEGF and inhibit angiogenesis caused by the high level expressions of VEGF, so as to prevent and stop retinal hemorrhage. This kind of VEGF inhibitors includes Macugen (pegaptanib sodium), Lucentis, VEGF-Trap, Avastin (bevacizumab), and AdPEDF etc. Particularly, Macugen and Avastin have been approved by US FDA and have come into the market.

### SUMMARY OF INVENTION

One aspect of the invention is to provide novel VEGF inhibiting fusion proteins FP7 and FP8, whose amino acid sequences are shown as SEQ ID No. 2 and 4.

FP7 consists of the 2^{nd} Ig-like domain of FLT-1, and the 3^{rd} and the 4^{th} Ig-like domains of KDR. FP8 consists of the 2^{nd} Ig-like domain of FLT-1 and the 3^{rd} Ig-like domain of KDR.

Another aspect of the invention is to provide the genes that encode the VEGF receptor fusion proteins FP7 and FP8, whose DNA sequences are shown as SEQ ID No. 1 and 3.

Another aspect of the invention is to provide expression vectors containing the aforementioned genes encoding the VEGF receptor fusion proteins FP7 and FP8. The expression vectors could be any common vectors in the field such as plasmid vector, phage, virus etc.

Another aspect of the invention is to provide a method for preparing VEGF inhibiting fusion proteins FP7 and FP8, which includes gene amplification of Flt-1 and KDR, preparation of the Flt-1 and KDR fragments by restriction enzyme digestion, ligation of the 2^{nd} Ig-like domain of Flt-1 and the 3^{rd} and 4^{th} Ig-like domain of KDR or ligation of the 2^{nd} Ig-like domain of Flt-1 and the 3^{rd} Ig-like domain of KDR; construction of a expression vector comprising the resulting ligation product; and transfection of the vector into host cells to produce the fusion proteins.

Another aspect of the invention is to provide a pharmaceutical composition for treating the angiogenesis related eye diseases, which contains a pharmaceutically effective amount of any one or combination of the VEGF receptor fusion proteins FP1, FP2, FP3, FP4, FP5, FP7, and FP8, such as FP7 and/or FP8, and optionally one or more of pharmaceutically acceptable carriers, particularlyone or more carriers that commonly used for ophthalmological therapeutics.

A further aspect of the invention is to provide use of VEGF inhibiting fusion proteins in the treatment of angiogenesis related eye diseases; wherein the aforementioned fusion proteins are the recombinant receptor fusion proteins that can bind VEGF, more specifically, said fusion proteins are any one or combination selected from the group consisting of FP1, FP2, FP3, FP4, FP5, FP6 described in the Chinese patent application No. 200510073595.4 "Anti-angiogenesis fusion proteins and applications thereof" and FP7 and FP8 described herein. Among them the amino acid sequences of the fusion proteins FP1, FP2, FP3, FP4, FP5, FP6 were disclosed in the aforementioned patent application No. 200510073595.4 (said application is incorporated into the present application by reference in its entirety). FP1 consists of the 2^{nd} Ig-like domain of FLT-1, the 3^{rd} Ig-like domain of KDR and human immunoglobulin Fc; FP2 consists of the 1^{st} Ig-like domain of KDR, the 2^{nd} Ig-like domain of FLT-1, the 3^{rd} Ig-like domain of KDR and human immunoglobulin Fc; FP3 consists of the 2^{nd} Ig-like domain of FLT-1, the 3^{rd} and 4^{th} Ig-like domains of KDR and human immunoglobulin Fc; FP4 consists of the 2^{nd} Ig-like domain of FLT-1, the 3^{rd} Ig-like domain of KDR, the 4^{th} Ig-like domain of FLT-1 and human immunoglobulin Fc; FP5 consists of the 2^{nd} Ig-like domain of FLT-1, the 3^{rd}-5^{th} Ig-like domains of KDR and human immunoglobulin Fc; FP6 consists of the 2^{nd} Ig-like domain of FLT-1 the 3^{rd} Ig-like domain of KDR, the 4^{th}-5^{th} Ig-like domains of FLT-1 and human immunoglobulin Fc. The amino acid sequences of the fusion proteins FP7 and FP8 are shown as SEQ ID No. 2 and 4 in the Sequence Listing. The aforementioned angiogenesis related eye diseases include, but not limited to, AMD, diabetic retinopathy, cystoid macular edema, diabetic macular edema, retinal vascular occlusion, angiogenesis related therapy failure such as laser coagulation, and surgical retinal transplantation.

Another aspect of the invention is to provide a method of treatment comprising administrating a pharmaceutically effective amount of FP3, FP7 and or FP8 to the patients in need thereof.

Another aspect of the invention is to provide a method for treating an angiogenesis related eye disease by administrating any one or combination selected from the group consisting of VEGF receptor fusion proteins FP1, FP2, FP3, FP4, FP5, FP6, FP7 and FP8 to a patient suffered from said disease; wherein said eye disease include, but not limited to, AMD, diabetic retinopathy, cystoid macular edema, diabetic macular edema, retinal vascular occlusion, angiogenesis related therapy failure such as laser coagulation, surgical retinal transplantation.

The fusion proteins can be used in a pure form, or as their solvates, or salts, or solvates of the salts. The fusion proteins described in the present invention include all these forms.

The VEGF receptor fusion proteins FP1, FP2, FP3, FP4, FP5 and FP6 described in the present invention have been described in the patent application No. CN200510073595.4, and can be prepared according to the method set forth in the application or other similar methods. FP7 and FP8 are novel compounds, the preparation of which can be carried out by the method similar to that described in the patent application No. CN200510073595.4, and has been described in detail in the following Example of the present invention.

The recombinant protein of the present invention obtained by gene recombination technique can be purified into pharmaceutical grade, and then mixed with conventional pharmaceutical acceptable carriers and/or other adjuvants depending on the requirements of the desired formulation, and prepared as appropriate pharmaceutical formulations according to common formulation methodologies. These formulations can be used for intravenous administration, intravitreal administration, intraperitoneal administration, subcutaneous administration, topical ocular administration such as eye drops; among other, preferred are solution formulation or lyophilized formulation that can be hydrated into solution before use.

In the formulations described in the present invention, the concentration of the fusion proteins are between 0.01 mg/mL to 1000 mg/mL; the specific dosage depends on the form of the formulation, clinical needs, etc. A general guideline suggests to administer 0.1mg - 3000mg, preferably 1mg-2000mg each treatment, once per day, or 2 days, or 3 days ,or 4 days, or 5 days, or 6 days, or a week, or 2 weeks, or 3 weeks, or a month, or several times per day, e.g. 2-3 times per day.

The aforementioned pharmaceutical acceptable carriers can be any carrier suitable in the form of formulations described in the present invention, preferably one or more of the following: sodium phosphate, sodium succinate, histidine, mannitol, trehalose dehydrate, polysorbate 20, sodium chloride, sucrose, tromeramol, cellulose, modified cellulose or lactose. The aforementioned formulations can contain pH formulation buffers such as phosphate, citrate, acetate, succinate, tromeramol (Tris), histidne or any combination thereof, with concentrations of 0 - 100 mM, e.g., 1 - 100 mM, and pH ranging from 3 - 9; and can also contain osmoregulators such as sodium chloride (concentration ranging from 0 to 200 mM, e.g. 1 - 100 mM), dextrose (concentration ranging from 0% to 50%, e.g. 1 -30%); and can contain stabilizer such as amino acids, glycerol, cyclodextrin, sucrose, trehalose dehydrate with concentrations 0% - 40%, preferably 1 - 30%; and can contain preservatives such as thimerosal, sodium bisulfite, benzyl alcohol, etc. In lyophilized formulations, excipients such as mannitol can be included with concentration of 0% to 40%, preferably 0.1% to 10%; whereas in solution formulations, surfactants such as polysorbate 20 or 80, SDS can be included with concentration of 0% to 2%, preferably 0.01 % to 1%. The formulations described in the present invention can also contain preservatives, stabilizers, solvents, cosolvents. Preferred solvents are water for injection, organic solvents such as ethanol, glycerol, or other isoosmotic solutions.

The present invention has surprisedly discovered that the fusion proteins herein were efficacious in treating various angiogenesis related eye diseases, such as AMD, diabetic retinopathy, cystoid macular edema, diabetic macular edema, retinal vascular occlusion, angiogenesis-related therapy failure such as laser coagulation, surgical retinal transplantation, in addition to great stability, safety, lack of side effect, and efficacy. Experiments have demonstrated the benefits of the present invention, with details shown in the following Examples.

### BRIEF DESCRIPTION OF THE FIGURES

Figure 1: Schematic drawings of the 8 fusion proteins described in the present invention;
Figure 2: Comparison of the VEGF binding affinities of the fusion proteins;
Figure 3: Effects of the fusion proteins on the retinal angiogenesis caused by ischemic atrophy;
Figure 4: Effect of the fusion proteins on choroidal neovascularization.

### BEST MODE CARRYING OUT THE INVENTION

The following examples further elucidate the present invention, but they should not be construed to limit the protection scope of the invention.

### Example 1: Construction of FP7

The fusion protein FP7 was constructed from the Flt-1 and KDR gene fragments of cDNA obtained by amplifing with the mRNA extracted from the HUVEC cells as template and the following primers:

| | |
|---|---|
| FLT-1 D2(F): | 5'-cctttcgtagagatgtacagtga-3' |
| FLT-1 D2(R): | 5'-tatgattgtattggtttgtccat-3' |
| KDR D3(F): | 5'-gatgtggttctgagtccgtctca-3' |
| KDR D3-4(R): | 5'-cggtgggacatacacaaccaga-3' |

Specific conditions are PCR amplificaton 30 cycels of denaturing at 95°C for 30 min, annealing at 56°C for 45 sec, extension at 72°C for 2 min, to obtain the PCR products of the Flt-1 and KDR Ig-like domains. The PCR products were cloned into the PCR2.1 plasmid with the TA cloning kit, followed by transformation into E.coli, picking out the white colonies, and culturing in the LB media overnight. The plasmids were extracted with the Qiagen plasmid purification kit, and then subjected to restriction enzyme digestion and sequencing confirmation. The cDNAs of the Flt-1 fragment, the KDR fragment and the IgG hinge region were ligated together by Sewing PCR. The EcoRI sites were introduced within the terminal primers. The PCR products were purified with the Qiagen kit following the restriction enzyme digestion with EcoRI, and then were cloned into the pcDNA3.1 plasmid. The recombinant plasmids were used to transform E.coli, followed by picking out positive colonies and culturing in the LB media overnight. The plasmids were extracted with the Qiagen kit and subjected to restriction enzyme digestion and squencing confirmation. The confirmed plasmids were used to transfect CHO cells, to obtain the cell lines to stably produce the fusion protein FP7. The amino acid sequence of FP7 is shown as SEQ ID No. 3, and the DNA sequence of FP7 is shown as SEQ ID No.1. Part of the hinge region was retained at the C terminal of the fusion protein.

### Example 2: Construction of FP8

The fusion protein FP8 was PCR amplified with the FP7 as template and primers flt-1D₂(F): 5'-cctttcgtagagatgtacagtga-3' and KDR D3-hing(R). The DNA sequence of the latter was 5'-aggtgctgggcacagtgggcatgtgtgagttttgtctttttcatggaccctgacaaatg-3'. It contains the complementary sequence of the 3rd Ig-like domain of KDR and partial sequence of the human IgG Fc hinge. The conditions of PCR amplification and gene cloning were the same as in the Example 1. At the end the PcDNA3.1 plasmids with the cloned FP8 were used to transfect CHO cells, and stable cell lines were obtained to produce the protein. The amino acid sequence of FP8 is shown as SEQ ID No.4, and its DNA sequence is shown as SEQ ID No.2.

### Example 3: Binding affinity experiments of the fusion proteins to VEGF

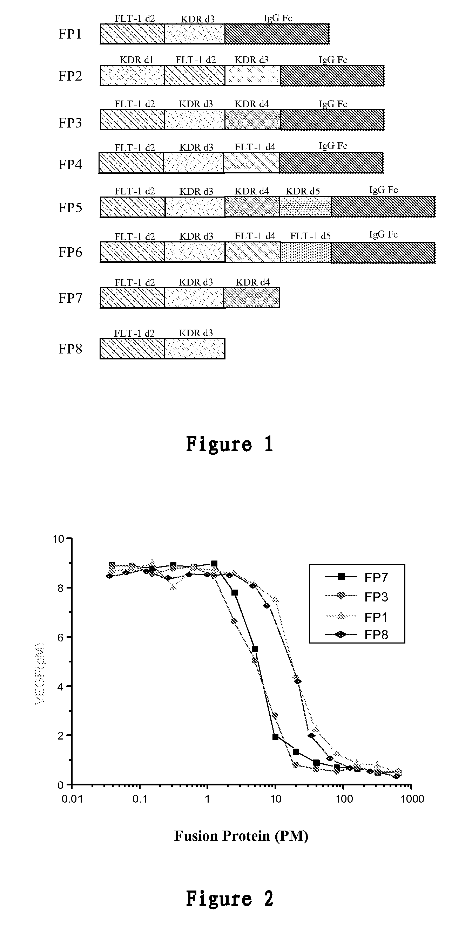

The present invention determined the affinity to VEGF of various fusion proteins by measuring the amount of VEGF. In the experiment, known amount of VEGF (10 PM) was added to a test tube, and then into this the same volume of various fusion proteins diluted to different degrees (as shown in Figure 2) were added, and upon mixing, incubated at 37°C for 1 hour. After 1 hour, amount of the free VEGF in the tube was determined using a VEGF assay kit (R&D systems). The assay results after analysis are shown in the Figure 2, which demonstrated FP1, FP3, FP7 and FP8 can bind effectively to VEGF with affinity shown as IC50 of 11.2PM, 4.3PM, 4.1PM and 11.2PM, respectively. This experiment has proved that FP3 and FP7 have similar in vitro affinity to VEGF, whereas FP8 and FP1 are similar but with lower affinity than the former two. The VEGF affinities of FP1-FP6 have already been described in the Example 3 of the Chinese patent application CN200510073595.4.

This experiment has further demonstrated that the amino acid sequence of the 4^{th} Ig-like domain of KDR can enhance the VEGF-binding capability of the fusion proteins.

### Example 4: Experiment results of the fusion proteins blocking angiogenesis caused by retinal ischemic atrophy

7-day new born mice were placed in an incubator with high oxygen partial pressure (75% ± 2%), temperature controlled at 23±2°C, and under sunlight. After several days, no neovascularization occured at the center of the retina; after 5 more days, the young mice were returned back into an incubator with normal oxygen partial pressure. Because of the hypoxia condition in the mice retina caused by the relatively lower oxygen partial pressure, angiogenesis growth was induced similar to those in diabetic retinopathy and other retinal degeneration caused by ischemic atrophy.

Using the models, 3 fusion proteins (FP1, FP3 and FP7) were assayed for their inhibitory effects on angiogenesis related to retinal ischemic atrophy.

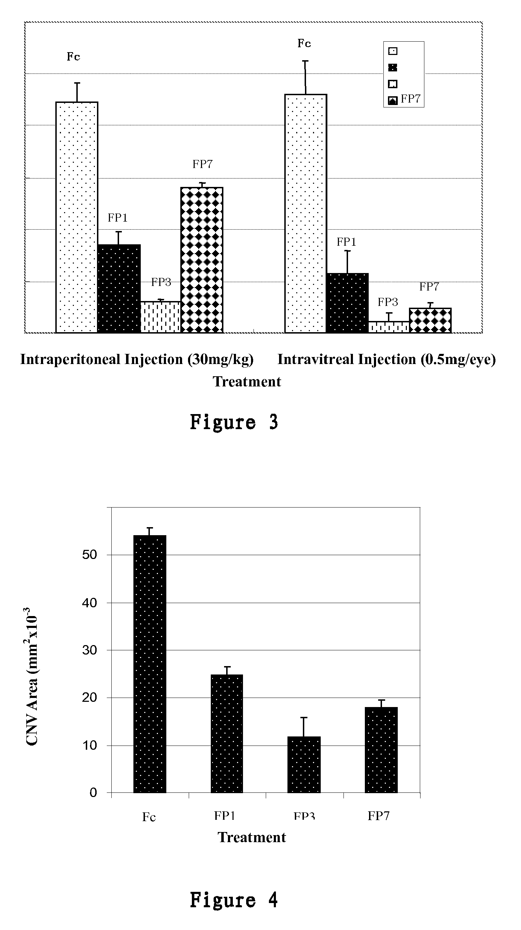

After in an incubator with high oxygen partial pressure for 5 days, young mice were returned to a normal oxygen partial pressure incubator. Young mice were divided into 5 groups with 10 each, and then after 1 day were administered intraperitoneally with 30 mg/kg of the fusion proteins, one injection per 2 days and 4 injections in total. Mice in the control group were injected with the same amount of Fc proteins. After treatment, 6 mice from each group were subjected to cardiac injection of fluorescent FAM. After 10 minutes, retinas of the young mice were taken to analyze the angiogenesis growth. Under fluorescence microscope, retinas were leveled and observed for angiogenesis and fluorescence leakage. For the other 4 mice of each group, the eyes were taken and embedded with paraffin , and then sliced and stained with H&E. Under microscope, numbers of the vascular endothelial cell nucleus were counted to analyze the effect of the fusion proteins on angiogenesis (Investigative Ophthalomogy Visual Science 43, 1994-2000, 2002). The results are shown in the Figure 3. The retinas of the young mice receiving the injection of Fc proteins have shown severe lesions. A lot of irregular vascular vessels can be observed on the choroid membranes. The retinas of the young mice receiving the injection of the fusion proteins have exhibited no obvious angiogenesis (as shown in the Figure 3). Among them, FP3 is the most effective, and FP1 and FP7 were also effective, in which lack of the Fc region probably has affected their stabilities in vivo.

In the meantime, we have tested the anti-angiogenesis effects of these fusion proteins administered intravitreally. In the same animal models, one day after returning to the normal oxygen partial pressure incubator, each eye was subjected to intravitreally injection of 0.5 µg of the fusion protein, only one treatment per animal. 7 days after treatment, retinas of the animals were collected and treated as described above. The effects of these fusion proteins on angiogenesis are shown in the Figure 3. The results have showed that these fusion proteins have exhibited significant inhibition to angiogenesis after intravitreal administration. Intravitreal injection has shown better efficacy than intraperitoneal injection. In the meantime, we have observed in these experiments that FP7 was similar to FP3, and both were better than FP1.In this experiment, FP7 does not need to enter blood circulation because of the intravitreal administration, therefore its low stability in circulation did not affect its efficacy.

### Example: 5 Effects of the fusion proteins on laser-induced choroidal neovascularization

According to published literature (American Journal Pathology 153, 1641-1646, 1998), we have established in rats a AMD model by laser-induced neovascularization in eyes. About 150 rats were divided into 4 groups, among them, 10 rats in the control group received subcutaneous injection of Fc proteins (20 mg/kg), whereas 10 rats in each test group received subcutaneous injections of 20 mg/kg FP1, FP3 and FP7 in total 5 times, at the day before and 3, 6, 9 and 12 days after the laser treatment. At the 15^{th} day after the laser treatment, the rats received 50 mg of fluorescence labeled dextran. After the eyes were taken out under anesthesia treatment, choroids were immediately separated and leveled or frozen to be sliced and then embedded with wax, in order to analyze the choroidal neovascularization (CNV) lesions. As shown in the Figure 4, the results have showed that the CNV areas were smaller in the rats receiving the fusion protein treatment than those from the control group, among which the efficacy of FP3 was better than that of FP1 or FP7. The inhibitory effects on the CNV of FP7 and FP1 were similar.

### Example 6: Application of the fusion proteins in treating eye diseases

These fusion proteins can be administered by appropriate formulations such as intravitreal or intravenous injections to treat various angiogenesis related eye diseases, including AMD, diabetic retinopathy, diabetic macular edema and central retinal vein occlusion. In the meantime, these fusion proteins can be applied by combining with other therapies, such as photosensitizer or laser therapy (photocoagulation) to reduce the risk of the therapy failure caused by angiogenesis. These fusion proteins can also be combined with surgery, for example, after retina transplantation, the surgery could fail because of angiogenesis. If receiving the treatment of these fusion proteins at the time of the surgery, the patient could improve the success rate of the retina transplantation. AMD patients could establish visual acuity baselines after routine eye examinations, and then receive these fusion proteins (e.g. FP3 or FP7) by intravitreal injection. After treatment, the patients would receive observations and examinations in a hospital to record the effects of these fusion proteins on AMD. Typically, the examinations occur once at the 1^{st}, 2^{nd}, 6^{th}, 14^{th}, 30^{th}, and 90^{th} days after treatment, respectively. In the meantime, the patients could receive multiple treatments of each injection every 2 to 8 weeks. Each injection dosage could range from 10 µg to 5 mg per eye.

From the clinical volunteers from the AMD, diabetic retinopathy, diabetic macular edema patients, the fusion proteins FP3, FP7 have been compared with the control experiments. (I) AMD

### 1. Efficacy of the fusion protein FP3

The control group received photocoagulation therapy; whereas the test group received FP3.

| Efficacy Comparison | | | | | |
|---|---|---|---|---|---|
| Group | Eye numbers | Significant Efficacious | Efficacious | Inefficacious | Ratio of efficacious |
| Test | 46 | 13 | 27 | 8 | 86.9% |
| Control | 40 | 4 | 19 | 14 | 57.5% |

The evaluation criteria were according to the efficacy assessment standards in the "Clinical research guidelines ofAMD treatment by the traditional Chinese medicines and novel medicines" issued by the Chinese Department of Health.
1) Significant efficacious: (i) significant symptom improvement of metamorphopsia etc; (ii) central vision is improved by at least 4 lines; (iii) minor shrinkage or obvious thinning of the central scotoma in the central visual field; (iv) obvious absorption of the macular exudation and hemorrhage, disappearance or obvious shrinkage of neuroepithelial and pigment epithelial cell detachments; (v) no progress or deceasing of retina angiogenesis assayed by fluorescein angiography; substantial reduction of fluorescein leakage; minor improvement of contrast sensitivity.
   Significant efficacious is scored if meeting 4 of above conditions,
2) Efficacious: (i) symptom improvement of metamorphopsia etc; (ii) central vision is improved by at least 2 lines; (iii) thinning of the central scotoma in the central visual field; (iv) partial absorption of the macular exudation and hemorrhage, some shrinkage of neuroepithelial and pigment epithelial cell detachments; (v) deceased fluorescein leakage in retina angiography; minor improvement of contrast sensitivity. Efficacious is scored if meeting 2-3 of the above conditions.
3) Inefficacious: not meeting efficacious criteria.

The results have shown that the fusion protein FP3 exhibited significant efficacy on treating the AMD.

### 2. Efficacy of FP7

The control group received photocoagulation therapy; whereas the test group received FP7.

| Efficacy Comparison | | | | | |
|---|---|---|---|---|---|
| Group | Eye numbers | Significant Efficacious | Efficacious | Inefficacious | Ratio of efficacious |
| Test | 45 | 16 | 21 | 8 | 82.2% |
| Control | 42 | 6 | 19 | 17 | 59.5% |

The evaluation criteria were the same as above.

The results have shown that the efficacy of the fusion protein FP7 on the treatment of AMD is no worse or better than the photocoagulation therapy.

### (II) Diabetic retinopathy

The control group received retina laser photocoagulation; whereas the test group received FP3.

| Efficacy Comparison | | | | | | | |
|---|---|---|---|---|---|---|---|
| Group | Number | Efficacy | | | | Z | P |
| | | SE(%) | E(%) | IE(%) | W(%) | | |
| Test | 107 | 43(40.2) | 44(41.1) | 20(18.7) | 0(0.0) | -1.963 | 0.050 |
| Control | 105 | 31(29.5) | 44(41.9) | 30(28.6) | 0(0.0) | | |

| Total Efficacy Comparison in Both Groups | | | | | |
|---|---|---|---|---|---|
| Group | Number | Efficacy | | Z | P |
| | | SE + E (%) | IE + W (%) | | |
| Test | 107 | 87 (81.3) | 20 (18.7) | -1.690 | 0.091 |
| Control | 105 | 75 (71.4) | 30 (28.6) | | |

Efficacy assessment criteria were according to the efficacy assessment standards of diabetic retinopathy in the "Clinical research guidelines of the traditional Chinese medicines and novel medicines". It includes 4 levels of efficacies: SE(significant Efficacious), E(Efficacious), IE(inefficacious), and W(worse).
1. Significant Efficacious (SE):
   1) Visual acuity is improved by at least 4 lines, or better than or equal to 1.0.
   2) Shown in ocular fundus examination, number of retina microaneurysm decreases from (+++) to (++), or from (++) to (+), or disappears from (+); retina hemorrhage decreases from (+++) to (+), or disappears from (++); exudation decreases from (+++) to (++), or from (++) to (+), or disappears from (+). At least two of microaneurysm, hemorrhage, and exudation shall meet the above-mentioned requirements.
   3) Shown by Fundus fluorescein angiography, average circulation time decreases significantly, macular edema alleviates significantly, retina capillary nonperfusion area decreases, blood vessel leakage reduces obviously. At least two of above criteria shall be satisfied, each with a level of improvement of at least 20%.
2. Efficacious (E):
   1) Visual acuity is improved by at least 2 lines.
   2) Shown in ocular fundus examination, number of retina microaneurysm decreases from (+++) to (++), or from (++) to (+), or disappears from (+); retina hemorrhage decreases from (+++) to (+), or disappears from (++); exudation decreases from (+++) to (++), or from (++) to (+), or disappears from (+). At least one of microaneurysm, hemorrhage, and exudation shall meet the above-mentioned requirements.
   3) Shown by fundus fluorescein angiography, average circulation time decreases, macular edema alleviates, retinal capillary nonperfusion area decreases, blood vessel leakage reduces obviously. At least two of above criteria shall be satisfied, each with a level of improvement of at least 10%.
3. Inefficacious (IE): do not meet the requirements shown above.
4. Worse (W):
   1) Visual acuity suffers loss of 2 lines or more.
   2) Ocular fundus color photography shows proliferative changes such as angiogenesis.
   3) Shown by fundus fluorescein angiography, retina capillary nonperfusion area increases, macular edema worsens, and blood vessel leakage increases.
   Note:
   Visual acuity examinations were performed using the standard visual chart (scale of 1). When less than 0.1, every 0.02 counts as one line.
   Fundus changes were assessed according to the ophthalmoscope or color photography, and microaneurysm was scored by the negatives of the fundus fluorescein angiography.
   (+) designates not many or countable microaneurysm, hemorrhage, or exudation; (++) designates a lot or difficultly countable microaneurysm, hemorrhage, or exudation; (+++) designates many, not countable microaneurysm, and significant hemorrhage and exudation, and fused together.
   When assessing efficacy, at least two of visual acuity, fundus changes, and fluorescein angiography have to be taken into account.
The results have shown that the fusion protein FP3 had the efficacy similar to that of the laser photocoagulation in the treatment of diabetic retinopathy.

### (III) Diabetic macular edema

The control group received laser photocoagulation; whereas the sample group received FP3.

| Efficacy Comparison | | | | | | | |
|---|---|---|---|---|---|---|---|
| Group | | Number of eyes | Acuity change | | | | P |
| | | | SE(%) | E(%) | IE(%) | χ2 | |
| March | Test | 42 | 19(45.24) | 18(42.86) | 5(11.90) | 0.261 | >0.05 |
| | Control | 45 | 20(44.44) | 18(40.00) | 7(15.56) | | |
| June | Test | 42 | 20(47.62) | 18(42.86) | 4(9.52%) | 0.64 | >0.05 |
| | Control | 45 | 18(40.00) | 21(46.67) | 6(13.33) | | |

| Group | | Number of eyes | Absorption of macular edema | | | P |
|---|---|---|---|---|---|---|
| | | | E(%) | IE(%) | χ2 | |
| March | Test | 42 | 30(71.43) | 12(28.57) | 0.83 | >0.05 |
| | Control | 45 | 28(66.22) | 17(37.78) | | |
| June | Test | 42 | 32(76.19) | 10(23.81) | 1.43 | >0.05 |
| | Control | 45 | 29(64.44) | 16(35.56) | | |

The efficacy assessment criteria were according to the "Clinical Research of Argon Laser Coagulation for Diabetic Retinopathy" by Ren Lianer (Journal of Ophthalmology, 1998, 7 (2): 86-88).

The visual acuity assessment criteria were according to the standard visual chart. Significant Efficacious (SE) was scored if visual acuity improved at least 2 lines after treatment; inefficacious (IE) was scored if visual acuity deteriorated at least 2 lines; no visual acuity change (E) was scored otherwise. If less than 0.1 before treatment, change of ≥±0.02 was assessed as visual acuity improvement or deterioration, otherwise it was considered no change.

The degrees of reduction of macular edema were assessed according to the fundus fluorescein angiography before or after the laser photocoagulation. Efficacious (E) was scored if there was no exudation in the macular area or the exudation had reduced at least a quarter of the area; Inefficacious (IE) was scored if the exudation had not reduced significantly or even increased.

The results have shown that intravitreal administrations of the VEGF receptor fusion proteins were more efficacious than the laser photocoagulation in both short period of time (3 months) or relatively long period of time (6 months) after treatment in the patients of macular edema.

### (IV) Retinal vein occlusion

The control group received laser photocoagulation; whereas the test group received FP3.

| Efficacy Comparison | | | | | |
|---|---|---|---|---|---|
| Group | Number of eyes | Significant Efficacious | Efficacious | Inefficacious | Ratio of efficacious |
| Test | 71 | 25 | 33 | 13 | 81.69% |
| Control | 68 | 10 | 43 | 15 | 77.94% |

Efficacy was assessed according to the "Evaluation of the retinal vein therapy" by Zhang Huirong (Journal of Beijing Medical College, 1982, 14: 118).

### Example 7: Preparation of the fusion protein FP3 in ophthalmic lyophilized formulation

First prepared formulation buffer (including 10 mmol/L histidine, 9% trehalose dehydrate, and 0.05% polysorbate 20, pH6.0), then thawed appropriate drug substance of FP3, diluted it with the formulation buffer to a required protein concentration (10 mg/mL), and thereby obtained the pharmaceutical composition. After sterilizing filtration, the required amount of the pharmaceutical composition was distributed into sterile vials (specification: 0.5 mL/2 mL) by pipettors or distributors, and the vials were applied with sterile butyl rubber stoppers (half way tightening). The vials were placed into a freeze drying lyophilizer, and then were freeze dried by setting up appropriate freeze drying procedures (including parameters of pre-chill, freezing, vacuum pump, time, temperature, vacuum degree, etc.). After the completion of the freeze drying, the stoppers were fully tightened, the vials were then taken out from the freeze drying lyophilizer and sealed with aluminum caps. After labeling, the vials can be placedd in paper boxes and stored under appropriate temperature.

### Example 8: Preparation of the fusion protein FP3 in solution for eye application

First prepared formulation buffer (including 5 mmol/L disodium phosphate, 5 mmol/L citric acid, 100 mmol/L sodium chloride, 20% sucrose, and 0.1% polysorbate 20, pH6.0), then thawed appropriate drug substance of FP3 and diluted it with the formulation buffer to required protein concentration (10 mg/mL) , and thereby obtained the pharmaceutical composition. After sterilizing filtration, the required amount of the pharmaceutical composition was distributed into sterile vials (5 mL/20 mL) by pipettors or distributors. The vials were applied with sterile butyl rubber stoppers tightly, sealed with aluminum caps, labeled on the vials, and stored in paper boxes under appropriate temperature.

### Example 9: Preparation of the fusion protein FP3 in solution for eye application

First prepared formulation buffer (including 10 mmol/L, sodium succinate, 9% trehalose dehydrate, and 0.1 % polysorbate 20, pH6.0), then thawed appropriate drug substance of FP3, diluted it with the formulation buffer to the required protein concentration (10 mg/mL), and thereby obtained the pharmaceutical composition. After sterilizing filtration, the required amount (≤100 µL) of the pharmaceutical composition was distributed into sterile vials (0.5 mL) by pipettors. The vials were applied with sterile butyl rubber stoppers tightly, sealed with aluminum caps, and labeled on the vials. The pharmaceutical composition was also introduced into syringes (1 mL, with gray rubber piston and 27-gauge needle) in the required amount(≤100 µL), rubber stoppers were applied at the pistons, and gray rubber covers and hard plastic sheathes were in turn applied at the needles. Then, the syringes were sealed with aluminum bags (in addition, a threaded plastic plunge and a white flange joint were enclosed in a separate aluminum bag). They were stored in paper boxes under appropriate temperature.

### Example 10: Preparation of the fusion protein FP1 in eye formulation

Other than using the fusion protein FP1 to replace the fusion protein FP3, all materials and methods are the same as in Example 9.

### Example 11: Preparation of the fusion protein FP2 in eye formulation

Other than using the fusion protein FP2 to replace the fusion protein FP3, all materials and methods are the same as in Example 9.

### Example 12: Preparation of the fusion protein FP4 in eye formulation

Other than using the fusion protein FP4 to replace the fusion protein FP3, all materials and methods are the same as in Example 9.

### Example 13: Preparation of the fusion protein FP5 in eye formulation

Other than using the fusion protein FP5 to replace the fusion protein FP3, all materials and methods are the same as in Example 9.

### Example 14: Preparation of the fusion protein FP6 in eye formulation

Other than using the fusion protein FP6 to replace the fusion protein FP3, all materials and methods are the same as in Example 9.

### Example 15: Preparation of the fusion protein FP7 in eye formulation

Other than using the fusion protein FP7 to replace the fusion protein FP3, all materials and methods are the same as in Example 9.

## Claims

1. A VEGF receptor fusion protein selected from FP7 and FP8 having the amino acid sequences as shown in SEQ ID No. 2 and 4, respectively.

2. Genes encoding the VEGF receptor fusion proteins FP7 and FP8, having the DNA sequences as shown in SEQ ID No. 1 and 3.

3. Expression vector comprising the gene encoding the VEGF receptor fusion proteins FP7 and / or FP8 of Claim 2.

4. A method for preparing the VEGF receptor fusion proteins FP7 and FP8 of Claim 1, comprising the procedures as follows: amplifying the Flt-1 and KDR genes, obtaining the Flt-1 and KDR fragments by restriction enzyme digestion of the resulting genes, ligating the 2^{nd} Ig-like domain of FLT-1 and the 3^{rd} and 4^{th} Ig-like domains of KDR, or ligating the 2^{nd} Ig-like domain of FLT-1 and the 3^{rd} Ig-like domain of KDR; constructing a expression vector of the fused gene; and transforming the vector into host cells to produce the fusion proteins.

5. A pharmaceutical composition used to treat eye diseases caused by abnormal angiogenesis, wherein the pharmaceutical composition comprises one or both of the VEGF receptor fusion proteins FP7 and FP8 in a pharmaceutically effective amount and optionally one or more pharmaceutically acceptable carriers.

6. A pharmaceutical composition as claimed in Claim 5, wherein the pharmaceutically acceptable carriers are any one or combination of water, mannitol, glycerol, ethanol, polysorbate, and glucose.

7. The use of VEGF receptor fusion protein in preparing a drug for treating angiogenesis related eye diseases, wherein the fusion protein is selected from one or combination of FP1, FP2, FP3, FP4, FP5, FP6, FP7, and FP8.

8. The use as claimed in Claim 7, wherein the angiogenesis related eye diseases are AMD, diabetic retinopathy, cystoid macular edema, diabetic macular edema, retinal vascular occlusion, angiogenesis-related therapy failure such as laser coagulation, and surgical retinal transplantation.

9. A method for treating an angiogenesis related eye disease, **characterized by** administration of one or combination selected from the group consisting of the VEGF receptor fusion proteins FP1, FP2, FP3, FP4, FP5, FP6, FP7 and FP8, such as FP3, FP7 and/or FP8 to a patient suffered from said disease.

10. The method as claimed in Claim 9, wherein the angiogenesis related eye disease is seleceted from the group consisting of AMD, diabetic retinopathy, cystoid macular edema, diabetic macular edema, retinal vascular occlusion, angiogenesis-related therapy failure such as laser coagulation, and surgical retinal transplantation.
